Europäisches Patentamt

European Patent Office

Office européen des brevets

(19)

(11) **EP 1 355 256 A2**

(12) # EUROPEAN PATENT APPLICATION

(43) Date of publication:
**22.10.2003 Bulletin 2003/43**

(51) Int Cl.7: **G06F 19/00**

(21) Application number: **03008184.8**

(22) Date of filing: **08.04.2003**

(84) Designated Contracting States:
**AT BE BG CH CY CZ DE DK EE ES FI FR GB GR
HU IE IT LI LU MC NL PT SE SI SK TR**
Designated Extension States:
**AL LT LV MK RO**

(30) Priority: **17.04.2002 JP 2002114492**

(71) Applicant: **Fairways Informatix, Inc.
Tokyo 104-0061 (JP)**

(72) Inventor: **Hoshino, Tyuji
Funabashi-shi, Chiba 274-0821 (JP)**

(74) Representative: **Becker Kurig Straus
Patentanwälte
Bavariastrasse 7
80336 München (DE)**

(54) **Computer method and devices for biomolecule analysis**

(57)     A method and a computing device are provided, which carry out an analysis of a π-π interaction without performing a nonempirical molecular orbital calculation, when analyzing a biomolecule by a computer.

In the computation method and the computing devices, when binding energy in the biomolecule is computed due to the noncovalent interaction, the magnitude of the π-π interaction between aromatic rings or between an aromatic ring and an a functional group is represented by a potential function in which a relative position including the direction of aromatic rings or of an aromatic ring and a functional group is a variable.

[FIG. 1]

EP 1 355 256 A2

**Description**

BACKGROUND OF THE INVENTION

(i) Field of the Invention

This invention relates to a method for analyzing a biomolecule by using a computer and to the computing devices executing the analysis. More particularly, it relates to a method and a device to evaluate an effect of a $\pi$-$\pi$ interaction by applying a potential function to the $\pi$-$\pi$ interaction, which is responsible for the intramolecular or intermolecular noncovalent bonding of biomolecules.

(ii) Description of the Related Art

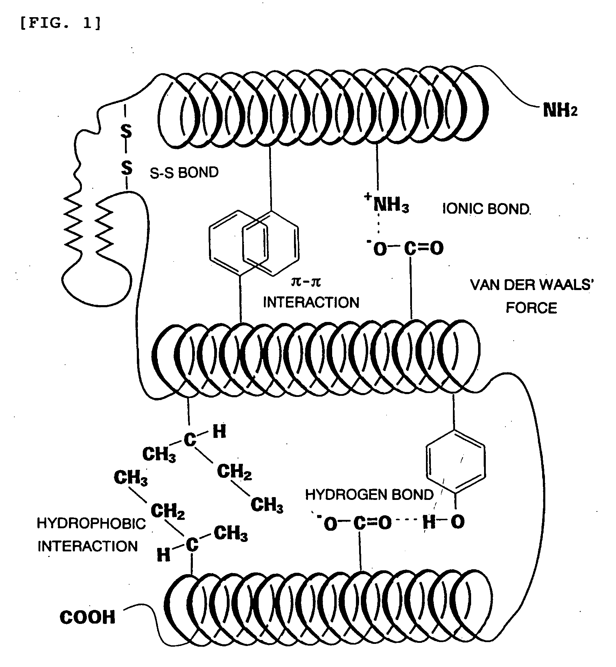

FIG. 1 is an explanatory view of a noncovalent interaction which is seen among the atoms in a protein molecule. As shown in FIG. 1, the interatomic noncovalent interaction of a biomolecule includes ionic bond, hydrogen bond, S-S bond, van der Waals force effect, $\pi$-$\pi$ interaction, hydrophobic interaction or the like.

In a computer simulation associated with a biomolecule, especially a computation based on a molecular force field method or molecular dynamics method, energy of a system is calculated separately as a contribution from the covalent bonds and a contribution from noncovalent interaction. The interatomic noncovalent interaction should be considered in the energy estimation of an intramolecular binding inside an isolated molecule as well as an intramolecular and intermolecular binding for complex molecules .

In a conventional computation based on the molecular force field method or molecular dynamics method, an interatomic noncovalent interaction in a biomolecule is evaluated as a sum of Coulomb force and van der Waals force only. Therefore, the effects of ionic bond, hydrogen bond, S-S bond, and dispersion force are incorporated, but the hydrophobic interaction and $\pi$-$\pi$ interaction are not incorporated. Although a computation for the spin correlation through a nonempirical molecular orbital calculation is inevitable for the evaluation of the $\pi$-$\pi$ interaction, the required computing time far exceeds a desirable and practical time range.

However, in addition to the Coulomb force and van der Waals force, the $\pi$-$\pi$ interaction has a significant influence on the noncovalent interaction which is seen among the atoms of a biomolecule, and it contributes significantly to the stabilization of the system.

It should be noted that, in this specification, the biomolecule refers to a protein, an enzyme, or the like in living matters as well as a chemical substances such as a medicine that can act thereon.

Furthermore, in this specification, the $\pi$-$\pi$ interaction refers to the intramolecular or intermolecular interaction between aromatic rings or between an aromatic ring and a functional group such as a CH group, an NH group, or an OH group.

As described above, the nonempirical molecular orbital calculation including the spin correlation is required to incorporate the $\pi$-$\pi$ interaction into the computation for analyzing a biomolecule. The problem is that the required calculation time far exceeds a desirable and practical time range.

SUMMARY OF THE INVENTION

This invention has an object to provide a method for analyzing a biomolecule by a computer and to provide the computing devices executing the analysis, so as to evaluate an effect of a $\pi$-$\pi$ interaction of the biomolecule by applying a potential function to the interaction, when analyzing the biomolecule by a computer or the computing devices.

In order to solve the problems described above, according to this invention, a method of analyzing a biomolecule by a computer comprises a step of evaluating an effect of a $\pi$-$\pi$ interaction by applying a potential function to an intramolecular or intermolecular $\pi$-$\pi$ interaction of the biomolecule.

In the method of analyzing the biomolecule by a computer, the effect of the $\pi$-$\pi$ interaction is calculated as an energy value.

In the method of analyzing the biomolecule by a computer, evaluation is made by adding a result obtained by calculating the effect of the $\pi$-$\pi$ interaction as the energy value to the result obtained by calculating the Coulomb force and van der Waals force.

In the method of analyzing the biomolecule by a computer, the potential function is separately adopted to the $\pi$-$\pi$ interaction between aromatic rings, and to the $\pi$-$\pi$ interaction between the aromatic ring and a functional group, such as a CH group, an NH group or an OH group.

In the method of analyzing the biomolecule by a computer, the $\pi$-$\pi$ interaction is evaluated with the Morse-type potential which is a function of a vector connecting the aromatic ring or the functional group with the other aromatic ring and is also a function of a vector normal to the plain made by the aromatic ring.

In the method of analyzing the biomolecule by a computer, the potential function $V_{\pi-\pi}$ between the aromatic rings, the potential function $V_{\pi\text{-CH}}$ between the aromatic ring and the CH group, the potential function $V_{\pi\text{-NH}}$ between the aromatic ring and the NH group, or the potential function $V_{\pi\text{-OH}}$ between the aromatic ring and the OH group, have the following potential functions:

$$V_{\pi-\pi} = V_{\pi-\pi}^{\text{vertical}} + V_{\pi-\pi}^{\text{parallel}}$$

$$V_{\pi-\pi}^{\text{vertical}} = D\left[\left\{1 - e^{-a(R-R_0)}\right\}^2 - 1\right]\left[1 - \left(\vec{n_i} \cdot \vec{n_j}\right)^2\right]\cos^2(\theta - \theta_0)$$

$$V_{\pi-\pi}^{\text{parallel}} = D\left[\left\{1 - e^{-a(R-R_0)}\right\}^2 - 1\right]\left(\vec{n_i} \cdot \vec{n_j}\right)^2\cos^2(\theta - \theta_0)$$

$$V_{\pi-\text{CH}} = D\left[\left\{1 - e^{-a(R-R_0)}\right\}^2 - 1\right]\cos^2(\theta - \theta_0)$$

$$V_{\pi-\text{NH}} = D\left[\left\{1 - e^{-a(R-R_0)}\right\}^2 - 1\right]\cos^2(\theta - \theta_0)$$

$$V_{\pi-\text{OH}} = D\left[\left\{1 - e^{-a(R-R_0)}\right\}^2 - 1\right]\cos^2(\theta - \theta_0)$$

Each symbol is as follows:

$Q_i(x_i, Y_i, z_i)$: central position of an aromatic ring i
$Q_j(x_j, y_j, z_j)$: central position of an aromatic ring or a functional group j

$$\vec{n_i}\left(n_{xi}, n_{yi}, n_{zi}\right):$$

normal vector of an aromatic ring i

$$\vec{n_i}\left(n_{xi}, n_{yi}, n_{zi}\right):$$

normal vector of an aromatic ring j

$$R = \left|\overrightarrow{Q_iQ_j}\right|:$$

variable

$$\theta = \text{arc cos}\left[\frac{\left(\vec{n_i} \cdot \overrightarrow{Q_iQ_j}\right)}{\left|\overrightarrow{Q_iQ_j}\right|}\right]:$$

variable

$D$, $a$, $R_0$, and $è_0$: constants.

In the method of analyzing the biomolecule by a computer, the constants are determined by performing nonempirical molecular orbital calculations with a simplified model system.

In the method of analyzing the biomolecule by a computer, the constants are shown in the following table 1:

Table 1:

| Parameters utilized in potential functions | | | | |
|---|---|---|---|---|
| kinds of potential functions | D (kJ/mol) | a | $R_0$ (Å) | $\theta_0$ (degree) |
| $V_{\pi\text{-}\pi}^{\text{vertical}}$ | 15.6962 | 1.3283 | 4.8284 | 0 |
| $V_{\pi\text{-}\pi}^{\text{vertical}}$ | 17.1660 | 1.3671 | 3.7771 | 27 |
| $V_{\pi\text{-CH}}$ | 3.5284 | 1.4095 | 3.9395 | 0 |
| $V_{\pi\text{-NH}}$ | 6.2360 | 1.2780 | 3.7579 | 0 |
| $V_{\pi\text{-OH}}$ | 9.3311 | 1.2019 | 3.5954 | 0 |

On the basis of the method of bilmolecule analysis according to any one of the above, a computer or the computing devices analyze a biomolecule.

BRIEF DESCRIPTION OF THE DRAWINGS

FIG. 1 is an explanatory view of a noncovalent interaction which is seen among the atoms in a protein molecule;
FIG. 2 shows a comparison of the calculated values by potential functions with the values by a nonempirical molecular orbital method; and
FIG. 3 shows a flowchart of the evaluation of an inhibition effect of a drug on a virus enzyme,.

DETAILED DESCRIPTION OF THE PREFERRED EMBODIMENTS

A ó bonding or a π bonding can be assigned for a covalent bond in biomolecules. In contrast, ionic bond, hydrogen bond, S-S bond, dispersion force effects are responsible for the noncovalent interaction. They can be estimated from the Coulomb force and van der Waals force. Although a π-π interaction is also involved in the noncovalent interaction, it cannot be estimated from the Coulomb force and van der Waals force. All of these noncovalent bonding effects contribute to the stabilization of a molecule or a complex of molecules.

This invention is directed to a method of analyzing a biomolecule by a computer, evaluating an effect of a π-π interaction by applying a potential function to an intramolecular or intermolecular π-π interaction of the biomolecule and also to the computing devices executing the above method.

An embodiment of this invention will hereinafter be described in detail.

First, the derivation of a potential function will be described. A potential function for evaluating the π-π interaction has a form of Morse-type formula respect to the direction and the distance of atomic groups constituting the biomolecule.

The atomic group constituting an aromatic ring or a functional group is extracted from all atoms constituting the biomolecule. Positions $Q_i(x_i, y_i, z_i)$, $Q_j(x_j, y_j, z_j)$ define the center points of the aromatic ring or the functional group. As to the functional group, the position of C, N or O atom is identical to the position $Q_j(x_j, y_j, z_j)$.

A line segment R binds an aromatic ring and an aromatic ring or binds an aromatic ring and a functional group, and an angle è is the angle between the line segment R and the normal vector on the plain of the aromatic ring. The line segment R and the angle è are variables expressed by the following Equations.

$$R = \left| \overrightarrow{Q_iQ_j} \right| :$$

variable

$$\theta = \arc \cos \left[ \frac{\left( \overrightarrow{n_i} \cdot \overrightarrow{Q_i Q_j} \right)}{\left| \overrightarrow{Q_i Q_j} \right|} \right] :$$

variable,

where all atoms of the aromatic ring are approximately located on the same plain. The normal vector with respect to the ring plain is expressed by the following notation. The functional group is treated as a particle, so that a normal vector is not defined.

$\overrightarrow{n_i}(n_{xi},n_{yi},n_{zi})$: normal vector of an aromatic ring i
$\overrightarrow{n_j}(n_{xj},n_{yj},n_{zj})$: normal vector of an aromatic ring j

A potential function $V_{\pi-\pi}$ of aromatic rings is the sum of the parallel and vertical components, which are each expressed by the following Equations. D, a, $R_o$, $è_o$ are constant parameters.

$$V_{\pi-\pi} = V_{\pi-\pi}^{\text{vertical}} + V_{\pi-\pi}^{\text{parallel}}$$

$$V_{\pi-\pi}^{\text{vertical}} = D\left[ \left\{ 1 - e^{-a(R-R_0)} \right\}^2 - 1 \right] \left[ 1 - \left( \overrightarrow{n_i} \cdot \overrightarrow{n_j} \right)^2 \right] \cos^2(\theta - \theta_0)$$

$$V_{\pi-\pi}^{\text{parallel}} = D\left[ \left\{ 1 - e^{-a(R-R_0)} \right\}^2 - 1 \right] \left( \overrightarrow{n_i} \cdot \overrightarrow{n_j} \right)^2 \cos^2(\theta - \theta_0)$$

Potential functions of an aromatic ring and a functional group, that is, a potential function $V_{\pi\text{-CH}}$ of an aromatic ring and a CH group, a potential function $V_{\pi\text{-NH}}$ of an aromatic ring and an NH group, and a potential function $V_{\pi\text{-OH}}$ of an aromatic ring and an OH group are expressed by the following Equations. D, a, $R_o$, $è_o$ are constant parameters.

$$V_{\pi-\text{CH}} = D\left[ \left\{ 1 - e^{-a(R-R_0)} \right\}^2 - 1 \right] \cos^2(\theta - \theta_0)$$

$$V_{\pi-\text{NH}} = D\left[ \left\{ 1 - e^{-a(R-R_0)} \right\}^2 - 1 \right] \cos^2(\theta - \theta_0)$$

$$V_{\pi-\text{OH}} = D\left[ \left\{ 1 - e^{-a(R-R_0)} \right\}^2 - 1 \right] \cos^2(\theta - \theta_0)$$

Next, a method to decide the above constant parameters D, a, $R_o$, $è_o$ will be described.
Nonempirical molecular orbital calculations (MP-2 / 6-311G (d, p)) are performed using a benzene ring and a benzene ring, a benzene ring and a $CH_4$, a benzene ring and an $NH_3$, or a benzene ring and an $OH_2$, respectively. Fitting the calculated energies with each potential function, constant parameters are decided by a least squares method.
The parameters decided above are shown in the following Table "Parameters utilized in potential functions".

Table 1:

| Parameters utilized in potential functions | | | | |
|---|---|---|---|---|
| kinds of potential functions | D (kJ/mol) | a | $R_0$ (Å) | $\theta_0$ (degree) |
| $V_{\pi\text{-}\pi}^{\text{vertical}}$ | 15.6962 | 1.3283 | 4.8284 | 0 |
| $V_{\pi\text{-}\pi}^{\text{vertical}}$ | 17.1660 | 1.3671 | 3.7771 | 27 |
| $V_{\pi\text{-CH}}$ | 3.5284 | 1.4095 | 3.9395 | 0 |
| $V_{\pi\text{-NH}}$ | 6.2360 | 1.2780 | 3.7579 | 0 |
| $V_{\pi\text{-OH}}$ | 9.3311 | 1.2019 | 3.5954 | 0 |

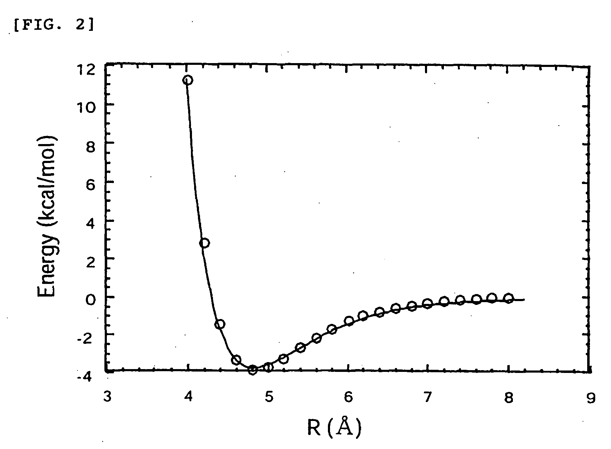

A comparison between the potential function using the parameters in Table 1 and the computed values by the nonempirical molecular orbital calculation (MP-2 / 6-311G (d, p)) is demonstrated in FIG. 2 "Comparison of calculated values by potential functions with computed values by nonempirical molecular orbit", in which another aromatic ring vertically approaches one aromatic ring (provided that è = 0 ). In FIG. 2, the horizontal axis indicates the distance between the two aromatic rings, and the vertical axis indicates energy values. Circular plots indicate the values calculated by performing the nonempirical molecular orbital calculation (MP-2 / 6-311G (d, p)), and a solid line indicates the values of the potential function $V_{\pi\text{-}\pi}$ (vertical).

The above potential functions are computer-programmed, so that energy of the $\pi$-$\pi$ interaction can be calculated by the computing devices.

The computation time of the $\pi$-$\pi$ interaction is compared between the computer-programmed potential function and the nonempirical molecular orbital calculation. For example, a computation finishing within one second by the potential functions requires 2021 seconds in the nonempirical molecular orbital calculation. This computation is executed in the situation where a benzene ring approaches another benzene ring up to 5.8 A with the normal vectors of the benzene rings being vertical to each other. MP-2 / 6-311G (d, p) is adopted in the nonempirical molecular orbital calculation, and $V_{\pi\text{-}\pi}$ (vertical) is employed for the potential functions. The CPU of the computer used is an Alpha chip of 833 MHz.

The above computations by the nonempirical molecular orbital method and by the potential functions indicate a good consistency and the computation time of the latter is extremely short. Similar advantage is expected for all other computations of the $\pi$-$\pi$ interaction.

An example of practical applications of the above method will be shown, in which the $\pi$-$\pi$ interaction of a biomolecule can be properly estimated to evaluate the medicinal efficacy of drugs.

Acquired immune deficiency syndrome (AIDS) is caused by human immunodeficiency virus (HIV). Anti HIV drugs include Ritonavir, Indinavir, Saquinavir and the like. These drugs inhibit the catalytic action of an HIV protease which is an enzyme indispensable for the replication of HIV. Their medicinal efficacies as inhibitors can be evaluated by computing the binding energy of an HIV protease and an inhibitor molecule. The potential functions of the $\pi$-$\pi$ interaction of biomolecules are used for the computation of the binding energy.

A brief summary of a computation method is as follows. An HIV protease structure is built in a computer. Then, an inhibitor is inserted in the reaction active site of an HIV protease. Many water molecules are added to mimic the inside of a living organism. Then, the binding energy of the HIV protease and the inhibitor molecule is computed.

The above computation is performed on the presumption that the whole system, comprising of a large number of water molecules, one HIV protease and one inhibitor molecule, keeps a steady structure in equilibrium condition at body temperatures.

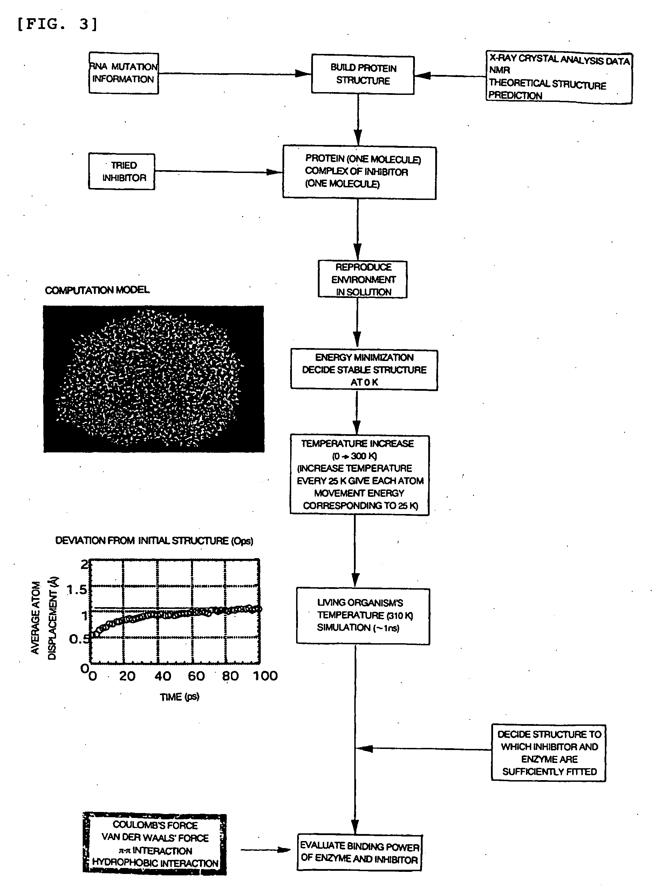

FIG. 3 "evaluation of an inhibition effect of a drug on a virus enzyme" is a flowchart illustrating the process to determine the structure in equilibrium condition and to evaluate the binding energy of an enzyme and an inhibitor.

In accordance with FIG. 3, an HIV-1 protease (equivalent to what is represented as a protein or an enzyme in FIG. 3) and Indinavir (an inhibitor) will appear as examples.

To determine the steady structure of the HIV-1 protease in equilibrium condition, first a tentative three-dimensional structure of the HIV-1 protease is constructed by either experimental data from the x-ray crystal analysis, NMR (nuclear magnetic resonance device), or other techniques or by theoretical approaches like a homology method. Then the structure of the HIV-1 protease is corrected in a computer on the basis of mutation information of RNA (ribonucleic acid).

Next, a sample inhibitor (e.g., Indinavir) is attached to form a so-called substrate-enzyme complex. Then, a large number of water molecules are added to solvate the complex.

Further, by using a molecular force field method, a stable state of the system, comprising of one HIV-1 protease, one Indinavir molecule, and a large number of water molecules, is determined with the energy minimization.

Next, a molecular dynamics simulation is performed to achieve the equilibrium at body temperatures. This simulation is performed for 0.1 n seconds at the temperatures and its average structure will be used as the structure in equilibrium condition for calculating the binding energy. The molecular dynamics simulation is performed because the structure of protein alters slightly at body temperatures from the experimentally obtained crystallographic structure.

At this point, the attachment of the HIV-1 protease and Indinavir is stable. Based on this steady state, the $\pi$-$\pi$ interaction between the HIV-1 protease and Indinavir will be estimated, in which the potential function of the $\pi$-$\pi$ interaction is used to evaluate their binding power.

Note that a "computation model" in FIG. 3 is the average structure of one HIV-1 protease, one Indinavir molecule and a large number of water molecules, resulting from a simulation done for 1 n seconds at body temperatures. In addition, "deviation from an initial structure (Ops)" in FIG. 3 is a structural change over time when the simulation is performed at a body temperature (310 K). Beyond 50 ps, the deviation from the initial structure is almost at a constant value. This means that the structure does not drastically change any more beyond 50 ps.

A detailed description will be given with an example of computing the binding state of Indinavir to the HIV-1 protease.

6846 water molecules (each constituted by three atoms) are added around one HIV-1 protease (constituted by 3110 atoms) and one Indinavir molecule (constituted by 92 atoms), thus the number of atoms amounts to 23734. When computing the $\pi$-$\pi$ interaction, a pair of aromatic rings or an aromatic ring and a functional group should be picked up. One of the pair must belong to the HIV-1 protease side, the other must belong to the Indinavir side. There are many such combinations of an aromatic ring and an aromatic ring or of an aromatic ring and a functional group, but about 105 of them have a noticeable binding effect in this example of the HIV-1 protease and Indinavir complex. A potential is computed in accordance with the potential functions mentioned above for each of the 105 and the computed potential energies are summed up.

Furthermore, the binding energy was calculated only by the Coulomb force and van der Waals force for comparison. The binding energy due to van der Waals force is computed using a Lennard-Jones type potential function. HIV-1 protease and Indinavir consist of 3110 and 92 atoms respectively, and 15 hydrogen bonds appear between them.

Table 2 "Evaluation of binding ability of an inhibitor to HIV-1 protease" shows the results of computing the binding energy with other inhibitors, i.e., Ritonavir and Saquinavir, and they are obtained in the same way described above.

Table 2:

| Evaluation of binding ability of an inhibitor to HIV-1 protease | | | |
|---|---|---|---|
| Drug | Ritonavir | Indinavir | Saquinavir |
| Number of formed hydrogen bonds | 10 | 15 | 10 |
| Binding energy of a drug per constituent atom calculated only by Coulomb's force and Van der Waals' force (kJ/mol) | 2.11 | 6.97 | 3.44 |
| Strength of a $\pi$-$\pi$ potential function (kJ/mol) | 39.4 | 52.3 | 23.9 |
| Comprehensive evaluation | $\Delta$ | $\bigcirc$ | $\Delta$ |

Table 2 enables a comprehensive evaluation of medicinal efficacies of these three anti HIV drugs as the inhibitors. In short, Indinavir is the most favorable, followed by Ritonavir, Saquinavir.

As described above, according to this invention, it is possible to provide a method for analyzing a biomolecule by a computer and to provide the computing devices, so as to evaluate an effect of a $\pi$-$\pi$ interaction of the biomolecule by applying a potential function to the $\pi$-$\pi$ interaction of the biomolecule.

**Claims**

1. A method of analyzing a biomolecule by a computer comprising a step of evaluating an effect of a $\pi$-$\pi$ interaction by applying a potential function to an intramolecular or intermolecular $\pi$-$\pi$ interaction of the biomolecule.

2. The method of analyzing the biomolecule by a computer according to claim 1, wherein the effect of the $\pi$-$\pi$ interaction is calculated as an energy value.

3.  The method of analyzing the biomolecule by a computer according to claim 2, wherein evaluation is made by adding a result obtained by calculating the effect of the π-π interaction as the energy value to a result obtained by calculating an effect of an intramolecular or intermolecular noncovalent bond of the biomolecule due to Coulomb force and Van der Waals force.

4.  The method of analyzing the biomolecule by a computer according to claim 1, wherein the potential function is separately adopted to the π-π interaction between an aromatic ring and an aromatic ring, and the π-π interaction between an aromatic ring and a functional group like a CH group, an NH group, or an OH group.

5.  The method of analyzing the biomolecule by a computer according to claim 4, wherein the π-π interaction is evaluated with Morse-type potential that is a function of a vector connecting the aromatic ring or the functional group with the other aromatic ring.

6.  The method of analyzing the biomolecule by a computer according to claim 4, wherein, as the potential function $V_{\pi-\pi}$ between the aromatic ring and the aromatic ring, the potential function $V_{\pi\text{-CH}}$ between the aromatic ring and the CH group, the potential function $V_{\pi\text{-NH}}$ between the aromatic ring and the NH group, or the potential function $V_{\pi\text{-OH}}$ between the aromatic ring and the OH group, the following potential functions are assigned:

$$V_{\pi-\pi} = V_{\pi-\pi}^{\text{vertical}} + V_{\pi-\pi}^{\text{parallel}}$$

$$V_{\pi-\pi}^{\text{vertical}} = D\left[\left\{1 - e^{-a(R-R_0)}\right\}^2 - 1\right]\left[1 - \left(\overrightarrow{n_i}\cdot\overrightarrow{n_j}\right)^2\right]\cos^2(\theta - \theta_0)$$

$$V_{\pi-\pi}^{\text{parallel}} = D\left[\left\{1 - e^{-a(R-R_0)}\right\}^2 - 1\right]\left(\overrightarrow{n_i}\cdot\overrightarrow{n_j}\right)^2\cos^2(\theta' - \theta_0)$$

$$V_{\pi-\text{CH}} = D\left[\left\{1 - e^{-a(R-R_0)}\right\}^2 - 1\right]\cos^2(\theta - \theta_0)$$

$$V_{\pi-\text{NH}} = D\left[\left\{1 - e^{-a(R-R_0)}\right\}^2 - 1\right]\cos^2(\theta - \theta_0)$$

$$V_{\pi-\text{OH}} = D\left[\left\{1 - e^{-a(R-R_0)}\right\}^2 - 1\right]\cos^2(\theta - \theta_0)$$

each symbol is as follows:

$Q_i(x_i, y_i, z_i)$: central position of an aromatic ring i
$Q_j(x_j, y_j, z_j)$: central position of an aromatic ring or a functional group j
$\overrightarrow{n_i}(n_{xi}, n_{yi}, n_{zi})$: normal vector of an aromatic ring i
$\overrightarrow{n_j}(n_{xj}, n_{yj}, n_{zj})$: normal vector of an aromatic ring j

$$R = \left|\overrightarrow{Q_iQ_j}\right|:$$

variable

$$f = \arc\cos\left[\frac{\left(\vec{n_i} \cdot \overrightarrow{Q_iQ_j}\right)}{\left|\overrightarrow{Q_iQ_j}\right|}\right] :$$

variable

D, a, $R_0$, and $è_0$: constants.

7. The method of analyzing the biomolecule by a computer according to claim 6, the constants are determined by performing nonempirical molecular orbital calculations with a simplified model system.

8. The method of analyzing the biomolecule by a computer according to claim 6, wherein the values in the following Table 1 are used for the constants:

Table 1:

| Parameters utilized in potential functions | | | | |
|---|---|---|---|---|
| kinds of potential functions | D (kJ/mol) | a | $R_0$ (Å) | $\theta_0$ (degree) |
| $V_{\pi\text{-}\pi}^{vertical}$ | 15.6962 | 1.3283 | 4.8284 | 0 |
| $V_{\pi\text{-}\pi}^{vertical}$ | 17.1660 | 1.3671 | 3.7771 | 27 |
| $V_{\pi\text{-}CH}$ | 3.5284 | 1.4095 | 3.9395 | 0 |
| $V_{\pi\text{-}NH}$ | 6.2360 | 1.2780 | 3.7579 | 0 |
| $V_{\pi\text{-}OH}$ | 9.3311 | 1.2019 | 3.5954 | 0 |

9. Computing devices which carry out a biomolecule analysis according to any one of claims 1 to 8.

[FIG. 1]

[FIG. 2]

[FIG. 3]

COMPUTATION MODEL

DEVIATION FROM INITIAL STRUCTURE (Ops)